(19) **Europäisches Patentamt / European Patent Office / Office européen des brevets**

(11) **EP 3 333 257 B1**

(12) **EUROPEAN PATENT SPECIFICATION**

(45) Date of publication and mention of the grant of the patent:
**20.11.2019 Bulletin 2019/47**

(21) Application number: **16832179.2**

(22) Date of filing: **14.07.2016**

(51) Int Cl.:
*C12N 5/10* (2006.01)  *C12N 15/85* (2006.01)
*C12Q 1/68* (2018.01)  *C12Q 1/66* (2006.01)
*C12Q 1/02* (2006.01)  *A61K 31/519* (2006.01)
*G01N 33/50* (2006.01)

(86) International application number:
**PCT/CN2016/089970**

(87) International publication number:
**WO 2017/020691 (09.02.2017 Gazette 2017/06)**

(54) **SCREENING AND USE OF PIPERIDINO PYRAZOLOPYRIMIDINE COMPOUND**

SCREENING UND VERWENDUNG EINER PIPERIDINO-PYRAZOLOPYRIMIDIN-VERBINDUNG

CRIBLAGE ET UTILISATION DE COMPOSÉ PIPÉRIDINO PYRAZOLOPYRIMIDINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.08.2015 CN 201510475276**

(43) Date of publication of application:
**13.06.2018 Bulletin 2018/24**

(73) Proprietor: **Institute of Basic Medical Sciences, Chinese Academy of Medical Sciences**
**Beijing 100005 (CN)**

(72) Inventors:
• **YANG, Jun**
**Beijing 100005 (CN)**
• **ZHOU, Fang**
**Beijing 100005 (CN)**
• **GONG, Shiqiang**
**Beijing 100005 (CN)**
• **WEI, Qingxia**
**Beijing 100005 (CN)**
• **ZHAO, Shuang**
**Beijing 100005 (CN)**

• **SI, Shuyi**
**Beijing 100005 (CN)**

(74) Representative: **Lermer, Christoph**
**LermerRaible Patent- u. Rechtsanwalts PartGmbB**
**Lessingstrasse 6**
**80336 München (DE)**

(56) References cited:
**WO-A1-2012/151153     CN-A- 1 867 666**
**CN-A- 105 062 976**

• **MARC HUMBERT ET AL: "Advances in Therapeutic Interventions for Patients With Pulmonary Arterial Hypertension", CIRCULATION, vol. 130, no. 24, 1 December 2014 (2014-12-01), pages 2189-2208, XP055531396, US ISSN: 0009-7322, DOI: 10.1161/CIRCULATIONAHA.114.006974**
• **LI, XIAOHUI;: "The Role and Related Molecular Mechanisms of BMP Signalling about Vascular Remodelling in Pulmonary Arterial Hypertension", CHINA DISSERTATION DATABASE (Wanfang Data, 8 October 2013 (2013-10-08), XP009509569,**

EP 3 333 257 B1

**Description**

**FIELD OF THE INVENTION**

The present invention relates the use of 1H-Pyrazolo[3,4-d]pyrimidine,1-phenyl-4-(1-piperidinyl)-as medicament for treatment of cardiopulmonary diseases.

**BACKGROUND OF THE INVENTION**

Pulmonary arterial hypertension (PAH), is a disease with increased pulmonary arterial resistance caused by the primary lesion of pulmonary arterioles. It is rare but with high mortality. The progressively elevated pulmonary vascular resistance finally leads to patient's right ventricular failure and death. The main pathological characteristic of PAH is severe pulmonary vascular remodeling including the fibrosis in neointima of small pulmonary arteries, the thickening of tunica media, the hyperplasia of the adventitia, plexiform lesion and occlusion of the arterial lumen, which result from the dysfunction of endothelium and proliferation of smooth muscle cells. Several new medicaments including prostacyclin and analogs, endothelin receptor antagonists and phosphodiesterase inhibitors, have been approved for marketing in the past 20 years, however, the 3-year mortality rate of the disease is still around 40%. And these treatment achieve their therapeutic effects mainly by reducing vasoconstriction. Therefore, it is necessary to develop a medicament which is able to improve vascular remodeling, to delay the progression of PAH, and to improve survival rate of the patients.

Optimized designing of the rational treatment regimens of PAH should be based on the thorough knowledge of cellular molecular mechanism of the disease. Research showed that the deficiency of the expression of Bone Morphogenetic Protein type II receptor (BMPRII) and reduced Id gene expression in PAH [Yang J at al Circulation Reserch 2008,102:1212-1221]. Recovery of physiological level of BMP signaling, especially the expression of Id gene in vascular cells is one of the new targets for the treatment of PAH which improves the pulmonary vascular remodeling [Yang J at al Am J Physiol Lung Cell Mol Physiol 2013 15:305(4):L 312-21].

Bone morphogenetic proteins (BMPs) are the members of TGFβ family, and play an important role in early development and differentiation, including the organogenesis of lung and vasculogenesis. In the presence of BMP ligands including BMP2, BMP4, BMP6, BMP9 and BMP10, BMPRII (with Ser/Ther kinase activity) phosphorylates the receptor-regulated Smad protein (R-Smad) through type I receptor. Among the effector genes which transcription are induced by BMP/Smad, the family members of inhibitor of DNA binding proteins (Id) are the most well known in the field. Id protein belongs to Helix-Loop-Helix (HLH) transcription factor, and competitively binds with the transcription factor with the structure of basic Helix-Loop-Helix (bHLH) so as to inhibit its activity. Id plays a role in cell growth, aging and differentiation. The result of experiment suggested that reduced expression of BMPR-II receptor and its downstream Id gene play a major role in the occurrence of PAH. To increase the concentration of BMP ligands is an effective method to enhance the expression level of downstream Id. A preliminary screening model for BMP2 up-regulator, i.e. A mouse cranial cell line MC3T3-E1 stably transfected with reporter plasmid comprising mouse bmp2 promoter and luciferase reporter gene, was developed firstly by the first inventor in 2002, in China. That model has been used in the discovery of anti-osteoporosis medicaments or their lead compounds to date. After more than 10 years of work at screening for BMP2 up-regulator, 2-acetylbenzothiophene and its analogues have been obtained and the research on anti-osteoporosis activity has been conducted. Recently, the up-regulator of BMP signaling was also proved to be a promising drug target for the treatment of PAH. Edda Spiekerkoetter and colleagues used C2C12-BRE cell line to screen the up-regulator of BMP signaling, and found that FK506 can treat and reverse PAH [Edda Spiekerkoetter at al The Journal of Clinical Investigation 2013,123(8):3600-3612]. However, due to the side effects, it is not approved for clinical application so far. C2C12-BRE cell line was derived from C2C12 cells which were stably transfected with a construct comprising BMP responsive element (BRE) of Id promoter region which linked to firefly luciferase reporter gene [Katagiri T at al Genes Cells 2002 7: 949-960]. The activity of luciferase induced by low dose (pm level) of BMP9 can be detected by luminescence. Firstly, screening model of BMP2 up-regulator is used in the present disclosure and the resulting BMP2 up-regulator is found to be able to increase BMP signaling and can be used in preliminary screening for PAH drug. Secondly, the screening model for up-regulator of Id protein expression, by which pulmonary vascular remodeling is specifically improved, is established, using the stem cell lines with the ability of cardiovascular differentiation.

One of the innovations of the present disclosure is the use of human embryonic stem cells in drug screening. Previously established screening system for BMP2 up-regulator was based on mouse osteosarcoma cell line, which has the defect of species differences with human cells.

The advantages of applying human embryonic stem cells to drug screening are that: 1.stem cells, which possess the ability to infinite proliferation, are suitable for the high throuput drug screening; 2. human embryonic stem cells could be differentiated into relevant cell types due to the pluripotency; 3. induced pluripotent stem cell technology can be used in screening according to individual differences between patients, which avoids the limitation that single cell line lacks effectivity to show of the differences between biological individuals and avoids the omissive and false screening [Willard VP at al Arthritis Rheumatol. 2014,66(11):3062-72]. For these reasons, it is a reasonable choice to replace the tumor cell by the cells which could be derived from any individual.

Human embryonic stem cells have the advantages that tumor cells do not have. However, whether such cells could be successfully applied to screen the PAH drug has not been reported so far. The inventors employ human embryonic stem cell techniques in the screening for PAH drug, and surprisingly find that the result of the assay is sensitive, stable and simple and has the advantages over current methods. According to the mechanistic study on the mainly affected signaling pathway in heritable and idiopathic PAH, a dual reporter gene element targeting Id1 gene, a key gene of BMP downstream signaling, was designed and inserted into Id1 genome to construct a recombinant vector. By transfecting the recombinant vector into a human embryonic stem cell line, the stable cell line CGMCC11091 was generated. With the expression of dual reporter gene as an indicator, the screening model for the compound which up-regulates Id1 expression was generated and could be used in the treatment of pulmonary arterial hypertension. Candidate compounds are tested with the desired technique steps set up at same time.

Humbert et al., "Advances in therapeutic interventions for Patients with Pulmonary Arterial Hypertension", Circulation., vol. 130, no. 24, Dec 9, 201, discloses on pages 2189-208. Nitric Oxide-cGMP pathway related to Sildenafil and Riociguat for the treatment PAH.

The innovation of the present invention is that a novel chemical structure type for the treatment of PAH, piperidino pyrazolopyrimidine compound, is identified from the screening method mentioned above. The compound has weak inhibitory effect on phosphodiesterase. The pyrimidinone core of sildenafil, which is similar with cGMP, competitively binds with phosphodiesterase type 5, inhibit the ring-opening and hydrolysis of cGMP. The piperidine ring of 1H-Pyrazolo[3,4-d]pyrimidine,1-phenyl-4-(1-piperidinyl)-, rather than ketone ring, determines its regulation on BMP signaling and the related activity.

## SUMMARY OF THE INVENTION

The object of the present disclosure is to establish a screening model for PAH medicament based on stem cell technology, and to identify the novel leading compound for the treatment of PAH by enhancing BMPRII signaling.

For this purpose, the present disclosure provides a cell line deposited under the number of CGMCC11091. The cell line is named as ID1-V-LUC hES, deposited at: China General Microbiological Culture Collection Center (CGMCC), address: Institute of Microbiology Chinese Academy of Sciences, 3, NO.1 West Beichen Road, Chaoyang District, Beijing, under the number of CGMCC No. 11091.

The present disclosure provides a model for screening PAH therapeutic compounds, characterized in that:
A dual reporter gene element was designed to target Id1 gene, a key gene of BMP downstream signal. The element was inserted into Id1 genome to construct a recombinant vector, which was used to transfect a human embryonic stem cell line. CGMCC11091 was generated with the Id1 promoter drove dual reporter gene stably expressed. After Stimulating with PAH candidate compounds, the cells were lysed with cell lysis buffer and their luciferase activity was detected by a method of reporter gene detection system, with the expression of dual reporter gene as an indicator. Thus the screening model for the compound which up-regulates Id1 expression was generated and could be used in the treatment of pulmonary arterial hypertension.

The present disclosure further provides a method of establishing the model, comprising the following steps:

(1) Retrieving the genomic sequence of Id1 gene from a database, and identifying exon 1 of Id1 gene as targeting locus, retrieving the BAC plasmids containing the complete sequence of Id1 gene (from 5Kb up stream of 5' to 5Kb downstream of 3').
(2) Bioinformatics analysis: using the computer program AOS to design and select the optimal loci for recombinant targeting, U5 and D3 loci which are located at 231bp downstream of exon 1 ATG of Id1 gene.
(3) Generating gene specific recombinant fragments by PCR:
The primers used to amplify recombinant fragment comprise the sequence of universal primer and the sequence of Id1 gene; inserting resistance-screening cassette, by electroporation, into a BAC plasmid which has induced recombinase activity, obtaining the BAC-Id1 plasmid carrying screening cassette.
(4) Construction of the intermediate vector:
Transforming the plasmid Psc101gbaA, which is based on the backbone of plasmid Psc101 and whose recombination is tetracycline(Tet)-inducible, into the clone of E.Coli carrying BAC plasmid comprising Id1 gene (BAC-Id1); inserting a DNA fragment, which contains Gateway R1/R2 sites and the positive (zeocin)/negative (pheS) selection markers of bacterial, into the U5/D3 loci of BAC-Id1 plasmid, in the first homologous recombination; constructing the intermediate vector through the gap-repair reaction between the plasmid pBR322 fragment carrying Gateway R3 and R4 sites and BAC-Id1, in the second homologous recombination.
(5) Construction of final targeting recombinant vector:
Obtaining the final targeting recombinant vector by the assembly of three plasmids based on Gateway system: the intermediate plasmid constructed in the present disclosure the pL1L2_BacT plasmid containing the targeting element and the pL3L4_DTA plasmid containing negative selection marker, finally, generating a high- copy targeting recombinant vector, Id1-Venus-Luc-MC1-DTA, by two Gateway switching reactions.
(6) Generating a screening cell line

By linearizing Id1-Venus-Luc-MC1-DTA plasmid with AsiSI, electroporating the resulting product into human embryonic stem cell H9 of passage 57, CGMCC11091 cell line was generated. It is a human embryonic stem cell line in which the expression of Venus-Luci dual reporter gene is drove by integrated Id1 promoter. CGMCC11091 cell line was differentiated to early mesoderm, and stimulated with pulmonary arterial hypertension candidate compounds. The cells were lysed with cell lysis buffer and their luciferase activity was detected by a method of reporter gene detection system, with the expression of dual reporter gene as an indicator. Thus the screening model for the compound which up-regulates Id1 expression was generated and could be used in the treatment of pulmonary arterial hypertension.

The present invention relates to the use of a compound obtained from the screening set up for the treatment of PAH, the compounds is: 1H-Pyrazolo[3,4-d]pyrimidine,1-phenyl-4-(1-piperidinyl)- Cas No:23000-46-6).

The use according to present disclosure also includes the screening for the following medicaments:

(1) A modulator up-regulating Bone Morphogenetic Protein (BMP) signaling;
(2) A modulator up-regulating the expression level of Bone Morphogenetic Protein type II receptor;
(3) A reagent improving pulmonary arterial hemodynamics and pulmonary vascular remodeling;
(4) A reagent promoting angiogenesis of endothelial cell;
(5) A medicament for the treatment of pulmonary arterial hypertension;
(6) A medicament for the treatment of cardiopulmonary disease.

The method for screening in the present disclosure mainly includes the following steps:

1. Screening BMP2 up-regulators having the ability to enhance BMP signaling and applying them to the preliminary screening for PAH medicaments: examining the effect of the compounds screened out by the model of BMP2 up-regulator on a myofibroblast cell line C2C12-BRE (Example 1).
2. Establishing and applying the stem cell-based secondary screening model for PAH drug.

(1) Targeting on Id gene, a key gene of BMP downstream signal, and constructing a targeting vector for Id1 gene in human embryonic stem cells (Example 2).
(2) Generating of stem cell-based screening model: By constructing dual reporter vector for screening Id1 up-regulator, a stable human embryonic stem cell line, ID1-V-LUC hES, was generated, in which the reporter vector is stably expressed (Example 3).
(3) Increasing the expression of Id1 by piperidino pyrazolopyrimidine compound in Human embryonic stem cell line ID1-V-LUC hES (Example 4).

3. Testing the therapeutic effect of the compound on PAH, using monocrotaline induced PAH rat model(Example 5).

The reagents used in the present disclosure are all commercially available.

## DESCRIPTION OF THE DRAWINGS

Figure1. The verification of BMP2 up-regulator by C2C12-BRE reporter system, wherein, A: OUR 1-19; B: BUR 1-12.
Figure 2. The flow chat of construction of targeting vector.
Figure 3. The design of Id1 gene targeting loci.
Figure 4. The flow chat of construction of intermediate vector.
Figure 5. The components used to construct the final vector.
Figure 6. Construction of final vector.
Figure 7. The final vector.
Figure 8. The comparison of luciferase activity and the expression levels of endogenous Id mRNA in the different clones stimulated by BMP4, wherein, A: Luciferase activity; B: the expression levels of the endogenous Id mRNA.
Figure 9. Clone ID1-V-Luc hES stimulated by BMP4 shows dose-dependent and time-dependent pattern.
Figure 10. The luciferase activity of ID1-V-Luc hES stimulated by different compounds.
Figure 11 The luciferase activity of ID1-V-Luc hES stimulated by piperidino pyrazolopyrimidine compound; wherein, y-axis: luciferase activity, x-axis: the concentration of the compound ($\mu$g/ml).
Figure 12. The effect of the piperidino pyrazolopyrimidine compound on promoting angiogenesis of endothelial cells.
Figure 13. The effect of administration of the compound on right ventricular systolic pressure of MCT-PAH rats.
Figure 14. The effect of administration of the compound on the survival rate of MCT-PAH rats. control: saline, MCT: monocrotaline, sildenafil: sildenafil as positive control, A: piperidino pyrazolopyrimidine compound.

## DETAILED DESCRIPTION OF THE INVENTION

The present invention is further illustrated by the following examples.

The experiments were conducted at State Key Laboratory of Medical Molecular Biology of Institute of Basic Medical Sciences, Chinese Academy of Medical Sciences, and natural and synthetic compounds samples were supplied by New drug (microorganism) screening laboratory of Institute of Medicinal Biotechnology, Chinese Academy of Medical Sciences.

1. Identifying the BMP2 up-regulators having the ability to enhance BMP signaling, and applying them to preliminary screening for PAH medicament.

**Example 1. Using C2C12-BRE cells to identify the BMP2 up-regulators which have the ability to enhance BMP signaling, and applying them to preliminary screening for PAH medicaments**

(1) Detection of the luciferase activity

A detection system of reporter gene, Luciferase Assay System (Promega), and EnVision 2104-0010 multi-function plate reader (Elmer) were used to detect the luciferase activity.

(2) Screening for small molecular compounds with BRE transcriptional activity.

C2C12-BRE cells (a modified myofibroblast cells, supplied by Department of Cellular Biology, Institute of Basic Medical Sciences, Chinese Academy of Medical Sciences) in logarithmic growth phase were trypsinized, and the cells were seeded in 96-well plate at 50,000 cells per well. When the cells were grown to 70-90% confluency, the old medium was removed, and the cells were gently washed once with PBS to avoid the interference of various active factors in the serum. Then, serum-free DMEM medium in same volume was added in each well. Each of total 31 compounds (comprising piperidino pyrazolopyrimidine compounds BUR1-12 and OUR1-19), at final concentrations of 2$\mu$g/mL and 10$\mu$g/mL was added to different wells, respectively. Triplicate replicates were used for each concentration of each compound. The well, containing only 1‰ DMSO was used as blank control. After the incubation of 18-24h at 37°C (5% CO2), the cells were lysed with cell lysis buffer CCLR (Promega), and the activity of luciferase was detected with the protocol according to the instruction of the detection system of reporter gene, Luciferase Assay System (Promega).

The luciferase activity from the cells stimulated with the compounds at different concentrations were calculated using the following formula:

$$\text{Regulation rate} = \frac{\text{Luciferase activity of compound-treated cells}}{\text{Luciferase activity of DMSO-treated cells}} \times 100\%$$

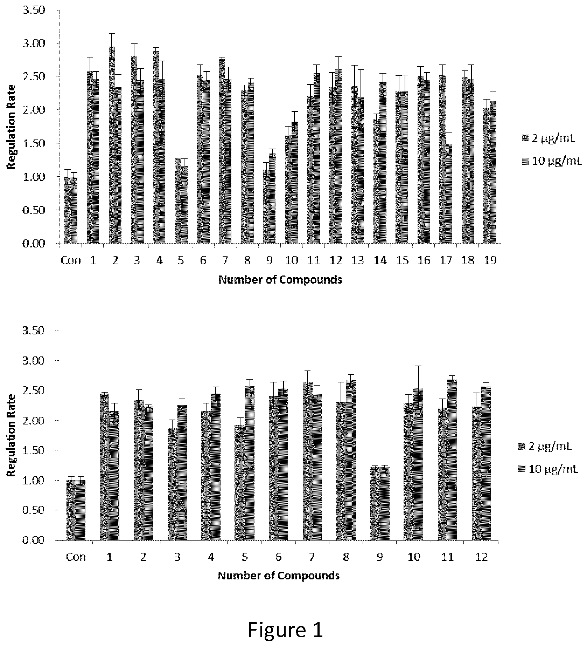

The results showed that most of the 31 compounds clearly have the function of BRE transcriptional activation ($\geq$1.5 fold), except BUR9, OUR1 and OUR9. The BRE transcription activity up-regulated by the 31 compounds are shown in Figure 1A and Figure 1B.

2. Establishment and application of the stem cell-based secondary screening model for PAH medicaments

Using Id gene, a key gene of BMP downstream signal, as a target, gene targeting vector was constructed; a vector containing dual reporter gene driven by Id promoter was constructed and a human embryonic stem cell line, in which the vector is expressed stably, was established.

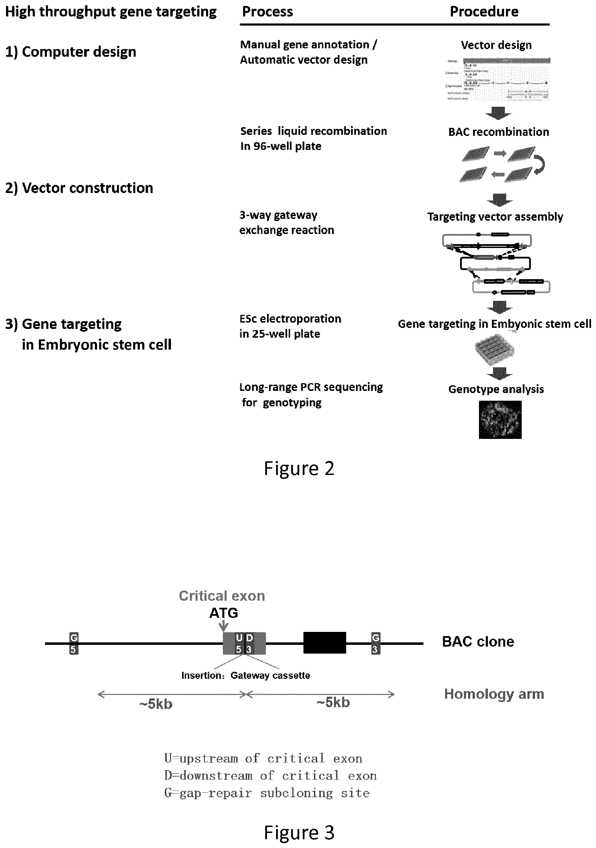

The targeting of Id gene in human stem cells was carried out by the GATWAY method. Firstly, a BAC plasmid, which has an appropriate length and contains the target gene, was retrieved from the constructed bacterial artificial chromosome library comprising whole human genome information. And then, the critical exon of the gene was identified through gene sequence analysis and functional domain analysis by computer program. Then the appropriate targeting locus was selected by computer program AOS (Array Oligo Selector). Then, the gene specific recombinant fragment was obtained using gene specific recombinant primers and high throughput PCR of 96-well plates. And then, a high efficient, Tet-inducible recombinant plasmid was transformed into the BAC clone in 96-well plate, then adding the purified recombinant fragments. In order to achieve the recombination between gene specific fragment and the corresponding BAC clone, so as to generate an intermediate vector library, two recombination reactions were carried out (In the first recombination, the cis element was inserted into single-copy BAC plasmid, in the second recombination, the BAC carrying the homologous arms with appropriate size was sub-cloned into another vector through gap-repair reaction). Then, the required elements were inserted into the intermediate carrier by 3-way Gateway system, so as to obtaining the final gene targeting vector. Finally, the purified gene targeting vector was electroporated into the embryonic stem cells in 25-well plate, and the gene targeting clones were obtained by negative selection. A flow chat of the construction of the high throughput gene targeting vector for stem cell and the procedure of obtaining the cell line are shown in Figure 2.

**Example 2: Construction of a gene targeting vector for Id1 gene of human embryonic stem cells**

1. The genome sequence of Id1 gene was retrieved from a database, and the exon 1 of Id1 gene was identified as targeting locus. Then the BAC plasmid (supplied by Sanger Research Institute) containing the complete sequence of Id1 gene (from 5Kb upstream of 5' to 5Kb downstream of 3') was retrieved.

2. Bioinformatics analysis: using the computer program AOS, the optimal nucleotide fragments for recombinant targeting were designed and selected in the candidate region as indicated by the arrow. Four recombinant targeting loci were designed (Figure 3), the U5 and D3 loci are located at 231bp downstream of exon 1 ATG of Id1 gene; G5 is located at about 6kb upstream of U5; G3 is located at about 4kb downstream of D3.

3. The procedure of generating gene specific recombinant fragment by PCR:
The primers used to amplify the recombinant fragment are composed of 70 bases. The 20bp at the 3'end of the primer is a universal primer of the resistance-screening cassette, the 50bp at the 5'end of the primer is the sequence of Id1 gene. High throughput primers were ordered in the form of 96-well plate, and the arrangement of primers corresponded to the gene specific BAC clones in the 96-well plate. Then the PCR reactions were finished in the96-well plate, and the PCR products (about 10kb) were purified by ultrafiltration, and then 2μg of purified DNA fragment was electroporated into BAC clone that has induced recombinase activity.

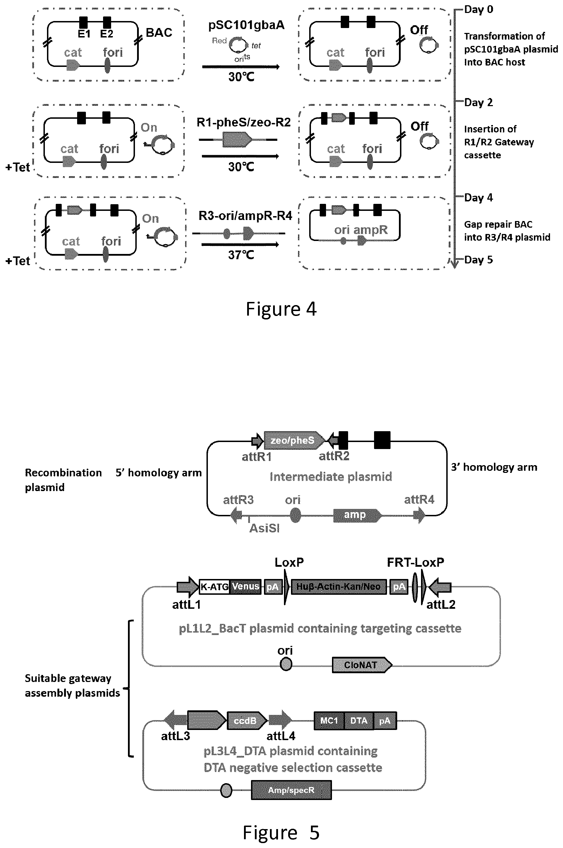

4. The procedure of construction of the intermediate vector:
The construction of the intermediate vector of high throughput Id1 gene was carried out in 96-well plate, mainly including three steps (Figure 4):

(1) Transformation of Bac

Plasmid Psc101gbaA (supplied by Sanger Research Institute), which is based on the backbone of Psc101 plasmid and is tetracycline(Tet)-inducible, was transformed into the clone of E.Coli carrying BAC plasmid comprising Id1 gene (BAC-Id1).

(2) Insertion of the sequence at upstream and downstream of the critical exon

A DNA fragment (supplied by Sanger Research Institute),which contains Gateway R1 and R2 sites and the positive (zeocin)/negative (pheS) selection markers of bacterial, was inserted into the U5 and D3 loci of BAC-Id1 plasmid.

(3) Gap repair

In order to remove BAC fragments flanking the homologous arms, the BAC fragments, which comprise the modified critical exon and 5kb of homologous arms at upstream and downstream of the critical exon, were sub-cloned into another vector by gap repair. The backbone of plasmid for gap repair is a linear plasmid based on pBR322 (supplied by the Sanger Research Institute). L3 and L4 Gateway sites of a high-copy plasmid which carries embryonic stem cells (ES) targeting negative selection marker(diphtheria A subtype driven by MC1 promoter, MC1-DTA) can be exchanged withR3 and R4 Gateway sites in the backbone. The final resulting vector was called as "intermediate vector". Different elements can be exchanged with the intermediate vector through attR1/R2 and R3/R4 sites of Gateway system, and finally, the desired targeting vector can be obtained.

5. The procedure of construction of final targeting recombinant vector:
The final targeting recombinant vector was obtained by the assembly of three plasmids based on Gateway system: the intermediate plasmid constructed the pL1L2_BacT plasmid containing the targeting elements and the pL3L4_DTA plasmid containing negative selection marker (Sanger Research Institute) (Figure 5). The present example includes two reactions based on Gateway based switching systems, each consisting of a set of two Gateway sites. The intermediate vector contains four Gateway sites (R1/R2 and R3/R4),and the vector containing targeting elements contains L1/L2 sites corresponding to R1/R2 sites, and the vector containing negative selection marker contains L3/L4 sites corresponding to R3/R4 sites.

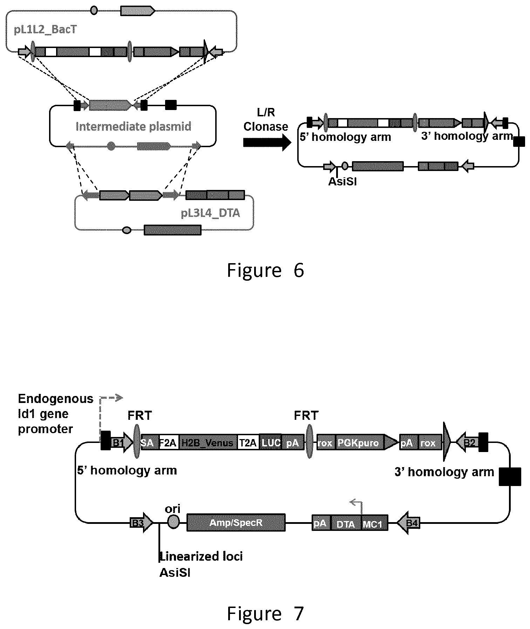

In the first switching system the negative selection marker (PheS) which islocated between R1/R2 sites of intermediate vector were exchanged with the dual reporter (Venus-Luc) system element(SA-T2A-H2B_venus-T2A-LUC-pA-promoter-puro-pA)(supplied by Sanger Research Institute)which is located between L1/L2 sites of the vector containing the targeting element(Figure 6). The second switching system was used to switch a fragment containing homology arms plus targeting element to a high-copy plasmid containing a mammalian negative selection gene (DTA) (supplied by Sanger Research Institute) by R3-L3 and R4-L4 reaction . R3 and R4 sites are locate at the ends of homologous arms of targeting vector, and the L3 and L4 sites flank a mammalian negative selection marker. Finally, a high-copy final vector was generated through the reaction of R3/R4 sites with L3/L4 sites, and named as Idl-Venus-Luc-MC1-DTA (Figure 7).

**Example 3: The generation of the Id gene targeting vector for human embryonic stem cells and the Id1-Venus-Luc dual reporter line.**

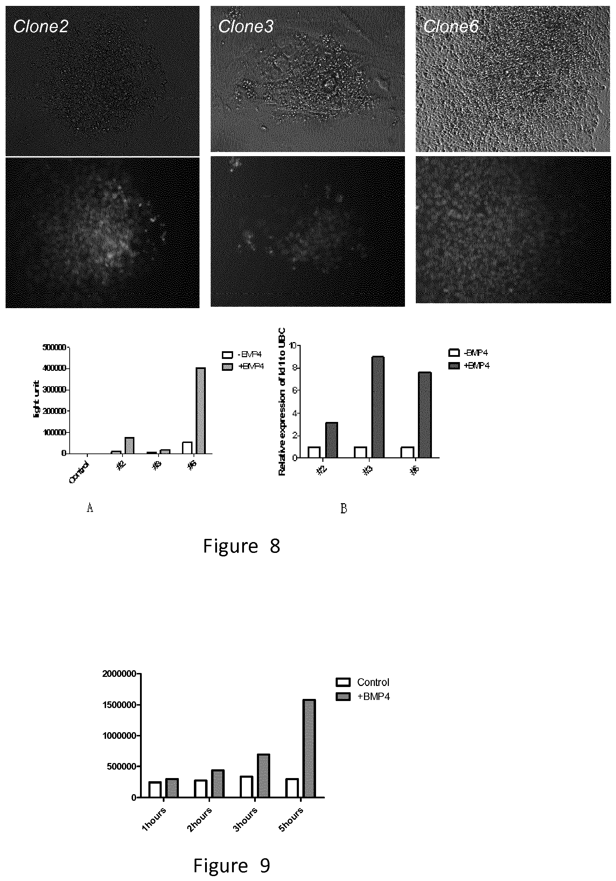

Id1-Venus-Luc-MC1-DTA plasmid was linearized with AsiSI, and then the resulting plasmid was electroporated into human embryonic stem cells (H9) of P57 (a commercially available human embryonic stem cells which could be passage to expand). At 37°C, CO2 5%, the cells were cultured on the DR4 MEF-feeder-cells (APPLIEDSTEMCELL, Inc., U.S.) with the medium containing serum replacement, and the medium was replaced by fresh medium daily. BioRad electroporation machine (ZAP: 250 V, uF 500, TC 8.6) was used for electroporation, then 19 clones were screened out using puromycin, and the clones with green fluorescence were picked out (the cells containing MC1-DTA plasmid cannot survive). The consistency between fluorescent activity and expression of endogenous Id gene was measured (Figure 8).

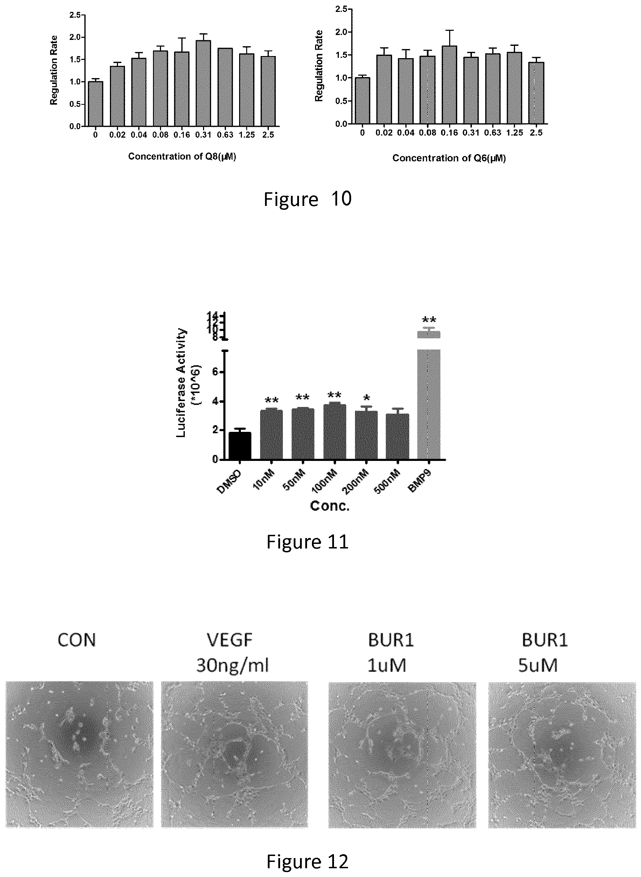

We found that after stimulation with same amount of BMP4, clone 2 had the consistency between luciferase activity and the mRNA expression of Id1 measured by QPCR. The luciferase activity increased over time (0, 1, 2 and 5 hours) after the stimulation with of BMP4. The effect of external stimulus on the expression of Id gene can be reflected maximally by expression level of luciferase. Therefore, clone 2 (named as ID1-V-LUC hES) was selected and was used to carry out the secondary screening for the compounds (Figure 9). ID1-V-LUC hES was differentiated toward early mesoderm, also the vascular cell with smooth muscle cell markers ($\alpha$ SMA, Calponin) were obtained. ID1-V-LUC hES differentiated cells were stimulated with BMP4 (Peprotech), and the effect of dose dependent activation was observed. The regulation rate of ID1-V-LUC reached 7.5 fold after 4 hours of stimulation with 6.25ng/mL of BMP4. Activities of 31 compounds were compared after 4 hours stimulation in two different concentrations (2$\mu$g/mL, 10$\mu$g/mL) (Figure 10). It was suggested that ID1-V-LUC hES model based on human embryonic stem cell line with Venus-Luci dual reporter gene driven by Id1 promoter was applicable for high throughput drug screening. ID1-V-LUC hES for the stem cell screening model was deposited at China General Microbiological Culture Collection Center under the number of: CGMCC No: 11091.

**Example 4: The application of the model based on human embryonic stem cell line with Venus-Luci dual reporter gene driven by Id1**

ID1-V-LUC hES were seeded in a 96-well plate and were grown to 80% confluency. The old medium was removed and the cells were washed with PBS, then mTeSR medium of human embryonic stem cell (Biowish.Co.,Ltd.) was added. The cells were starved for 6 hours prior to incubating with the compound: 1H-Pyrazolo[3,4-d]pyrimidine, 1-phenyl-4-(1-piperidinyl)- (Cas No:23000-46-6). The compound was serially diluted to 5, 2.5, 1.25, 0.625, 0.3$\mu$g/mL in above-mentioned medium, and sequentially added in the 96-well plate in which the stem cells of the model were seeded. Triplicate replicates were used for each concentration. At the same time, the well in which the medium containing 0.1‰ DMSO was added as blank control. After the incubation of 18-24 h at 37°C (5% CO2), the cells were lysed with cell lysis buffer CCLR (Promega), and the luciferase activity were detected using the method according to the instruction of the detection system of reporter gene, Luciferase Assay System (Promega).

The activity of luciferase resulting from the stimulation of the compounds at different concentrations was calculated using the following formula:

$$\text{Regulation rate} = \frac{\text{Luciferase activity of compound-treated cells}}{\text{Luciferase activity of DMSO-treated cells}} \times 100\%$$

The result showed that expression level of luciferase of the stem cells of the model is up-regulated by piperidino pyrazolopyrimidine compound in dose-dependent. The peak expression level, which reach 3.31 fold, is obtained when the concentration of the compound is 2.5μg/mL (Figure 11). It is demonstrated that this compound has the ability to up-regulate the expression of Id1 gene.

**Example 5: The effect of piperidino pyrazolopyrimidine compound on promoting the angiogenesis of endothelial cells.**

The endothelial cells were grown in complete culture medium (1%FBS in SFM) to confluency. One day before the experiment of angiogenesis, the medium was replaced by 0.2%FBS SFM to starve the cells for 16 hours. One the day before the experiment, Matrigel (Corning, 10mg/ml) was put on ice overnight for thawing, and on the day of experiment, Matrigel was added in the wells of the pre-chilled 96-well plate (50μl per well) and was distributed evenly in the wells. Then the plate was put in the incubator and incubated at 37□ for 30mins. Afterwards, the medium for starvation was removed and the cells were washed twice with DPBS, then Accutase enzyme was added to digest the cells for 1 minute. Later on, the SFM medium for starvation was added to resuspend the cells, and the cells were gently pipetted to form single cell suspension and centrifuged in room temperature at 1000rpm for 4 mins. The cells were resuspended in SFM medium for starvation and counted. Then, the cells were seeded in $1 \times 10^4$ cells per well, and were gently shook to distributed evenly before putting in the incubator at 37□. The angiogenesis was observed within 2-8 hours.

As shown in Figure 12, BUR1 (1μM and 5μM) treated ECs have significantly increased ability of angiogenesis, compared with control VEGF treated group; and BUR1 (1μM and 5μM) treated ECs have comparable ability of angiogenesis as that of VEGF treated ECs.

**Example 6: The effect of piperidino pyrazolopyrimidine compound on monocrotaline induced PAH rat model (MCT-PAH).**

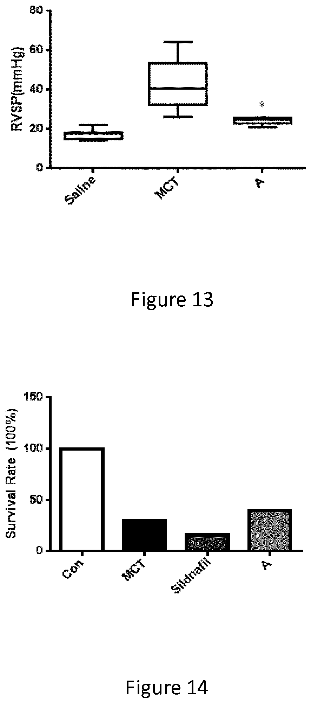

*In vivo* experiments with MCT-PAH rats: Monocrotaline (MCT, 55mg/kg) or saline were administrated subcutaneously in single dose to male Sprague-Dawley rats (supplied by Nation Institutes for Food and Medicament Control). Three weeks after the injection of MCT, 4.5mg/kg of piperidino pyrazolopyrimidine compound (A), saline or positive control (Sildenafil) were administrated respectively to the rats by intragastric gavage. Two weeks after the administration, the right ventricular systolic pressure (RVSP) was measured and the survival rate was calculated with the data of RVSP recorded in BL-420E system (Figure 13). It was suggested that the pulmonary arterial pressure of MCT-PAH rats decreased significantly and the survival rate of the rats with MCT-PAH increased obviously, after the administration of piperidino pyrazolopyrimidine compound (Figure 14).

| PCT | Print Out (Original in Electronic Form) | |
|---|---|---|
| 0-1<br>0-1-1 | **Form PCT/RO/134 Indications Relating to Deposited Microorganism(s) or Other Biological Material (PCT Rule 13bis)**<br>Prepared Using | |
| 0-2 | **International Application No.** | PCT/CN2016/089970 |
| 0-3 | **Applicant's or agent's file reference** | PCT201605 |

| 1<br>1-1 | **The indications made below relate to the deposited microorganism(s) or other biological material referred to in the description on:** | |
|---|---|---|
| 1-2 | **page** | 3 |
| | **line** | 10 |
| 1-3 | **Identification of deposit** | |

(continued)

| PCT | | Print Out (Original in Electronic Form) | |
|---|---|---|---|
| 1-3-1 | Name of depository institution | | **CGMCC China General Microbiological Culture Collection** |
| 1-3-2 | Address of depository institution | | **Center** |
| | | | **Institute of Microbiology, Chinese** |
| 1-3-3 | Date of deposit | | **Academy of Sciences, Mailbox 2714,** |
| 1-3-4 | Accession Number | | **Beijing 100080, China** |
| | | | **23 July 2015 (23.07.2015)** |
| | | | **CGMCC 11091** |
| 1-4 | **Additional Indications** | | **taxonomic designation: human embryonic stem cell ID1-V-LUC hES** |
| 1-5 | **Designated States for Which Indications are Made** | | **All designations** |
| | **FOR RECEIVING OFFICE USE ONLY** | | |
| 0-4 | **This form was received by the international Bureau on:** (yes or no) | | |
| 0-4-1 | Authorized officer | | |
| | **FOR INTERNATIONAL BUREAU USE ONLY** | | |
| 0-5 | **This form was received by the international Bureau on:** | | |
| 0-5-1 | Authorized officer | | |

**Claims**

1. Use of the compound 1-phenyl-4-(1-piperidinyl)-1H-Pyrazolo[3,4-d]pyrimidine for the manufacture of a medicament for the treatment of pulmonary arterial hypertension.

**Patentansprüche**

1. Verwendung der Verbindung 1-Phenyl-4-(1-piperidinyl)-1H-pyrazolo[3,4-d] Pyrimidin zur Herstellung eines Arzneimittels zur Behandlung von pulmonaler arterieller Hypertonie.

**Revendications**

1. Utilisation du composé 1-phényl-4-(1-pipéridinyl)-1H-pyrazolo [3,4-d] pyrimidine pour la fabrication d'un médicament pour le traitement de l'hypertension artérielle pulmonaire.

Figure 1

| High throughput gene targeting | Process | Procedure |
|---|---|---|
| 1) Computer design | Manual gene annotation / Automatic vector design | Vector design |
| 2) Vector construction | Series liquid recombination In 96-well plate | BAC recombination |
| | 3-way gateway exchange reaction | Targeting vector assembly |
| 3) Gene targeting in Embryonic stem cell | ESc electroporation in 25-well plate | Gene targeting in Embyonic stem cell |
| | Long-range PCR sequencing for genotyping | Genotype analysis |

Figure 2

Critical exon
ATG

BAC clone

Insertion: Gateway cassette

~5kb      ~5kb      Homology arm

U=upstream of critical exon
D=downstream of critical exon
G=gap-repair subcloning site

Figure 3

Figure 4

Figure 5

Figure 6

Figure 7

Figure 8

Figure 9

Figure 10

Figure 11

Figure 12

15

Figure 13

Figure 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2016089970 W

**Non-patent literature cited in the description**

- **YANG J.** *Circulation Reserch,* 2008, vol. 102, 1212-1221
- **YANG J.** *Am J Physiol Lung Cell Mol Physiol,* 2013, vol. 15 (4), L 312-21
- **EDDA SPIEKERKOETTER.** *The Journal of Clinical Investigation,* 2013, vol. 123 (8), 3600-3612
- **KATAGIRI T.** *Genes Cells,* 2002, vol. 7, 949-960
- **WILLARD VP.** *Arthritis Rheumatol.,* 2014, vol. 66 (11), 3062-72
- **HUMBERT et al.** Advances in therapeutic interventions for Patients with Pulmonary Arterial Hypertension. *Circulation,* vol. 130 (24), 2189-208